# EUROPEAN PATENT APPLICATION

(11) **EP 3 150 253 A1**
(43) Date of publication of application: **05.04.2017**
(21) Application number: 16190448.7
(22) Date of filing: 23.09.2016
(51) Int. Cl.: A61N 2/02, A61N 2/00

(54) **MAGNETOTHERAPY APPARATUS**

(30) Priority: 01.10.2015 IT UB201577454 U
(71) Applicant: SIXTUS ITALIA S.r.l., 59100 Prato (PO) (IT)
(72) Inventor: MARRUCCI, Massimo, 59100 Prato (PO) (IT)
(74) Representative: Ponzellini, Gianmarco

(57) **Abstract**

The present invention refers to a magneto therapy apparatus (1) comprising at least one magneto therapy terminal (2) configured for and being destined to be worn by a patient and to be placed in proximity of a body surface of the patient for performing a magneto therapy cycle, and a control device (3) for supplying command signals to the magneto therapy terminal (2). The apparatus (1) comprises at least one control unit (11) for supplying said command signals and an energy storage element (12) fixedly or removably connectable or connected to the magneto therapy terminal (2) for providing an electric supply to said at least one magneto therapy terminal (2), the control device (3) comprising a wireless communication module (21) and being configured for wirelessly communicating with a mobile device (23). The mobile device (23) is provided with a mobile application configured for supplying command signals to the control device (3) for performing a magneto therapy cycle.

## Description

The object of the present invention is a magneto therapy apparatus. The apparatus, object of the invention, is adapted to be used by private users and also in hospitals, orthopedic departments, postoperative rehabilitation centres, gymnasiums, medical offices, sports clubs, private physiotherapy centres, private pain treatment centres, nursing homes.

As it is known, the magneto therapy is used for treating some diseases by pulsed magnetic fields at a low frequency and low or high intensities. The magneto therapy has long met the largest consensus in the international scientific communities especially with reference to the chronic and degenerative diseases and particularly for treating the osteoporosis, and the delays in the bone consolidation. In other words, the magneto therapy uses the effects of a magnetic field generated by electric currents for accelerating repairing processes (particularly with reference to the bones) or for obtaining analgesic effects. The magneto therapy can be put in relation with many effects: the piezoelectric effect, the effect of orienting the collagen, the stimulation of the calcic deposition (Barker - Lunt 1983, Bassett - Pawluk - Pilla 1974, Bassett - Valdes - Hernandez 1982). The ideal treatment, according to the latest researches, provides, for the osteoporosis, extended treatments of some hours and more; there is a substantial regression of the pain symptomatology already from the sixth session.

To date, there are known apparatuses for performing the magneto therapy.

The magneto therapy apparatuses are usually cumbersome and are installed in dedicated spaces, such as physiotherapy centres, to which the patient goes for being subjected to a magneto therapy treatment. The known magneto therapy apparatuses exhibit a command unit and cables for being connected to suitable coils capable of generating magnetic fields; such devices are positioned at the body side of the patient destined to be treated. The control, treatment settings and calibrations of the operative parameters of the apparatus are usually performed by a physician or by a person directly assigned to the apparatus itself.

However, the known apparatuses have some disadvantages.

During the operation of the apparatus, the patient is on a bed or a chair and is constrained in proximity of the apparatus. Under such condition, the activities that the patient can perform during the magneto therapy cycle are limited; particularly, the patient cannot go away from the apparatus during the operation of the same. The known apparatuses can be used only in some specialized centres because they require to be supervised by a physician or a professional. Moreover, as hereinbefore discussed, the known apparatuses are cumbersome and heavy and therefore cannot be easily moved. Anyway, generally, the magneto therapy apparatuses are rather expensive and therefore their diffusion is limited to specialized centres and consequently are used only by a small fraction of the persons which instead could potentially need them.

Moreover, there are some known magneto therapy apparatuses for a domiciliary use.

In light of the above, the main object of the present invention consists of solving one or more of the problems found in the prior art. An object of the present invention consists of providing an apparatus enabling to comfortably perform magneto therapy cycles also at home. A further object of the invention consists of providing an apparatus enabling a physician to control the assigned protocols are correctly performed by a patient, and the effectiveness thereof, having therefore the possibility of intervening to modify the therapeutic protocol for making it more effective. Moreover, it is an object of the present invention to provide an apparatus having a simple and intuitive operation. Another object of the present invention consists of providing a magneto therapy apparatus whose operation can be managed also by an unskilled person, for example by the patient himself/herself performing the treatment. An additional object of the invention consists of providing a magneto therapy apparatus which is compact, lightweight and/or wearable. A further object of the present invention consists of meeting the requirements of having a device equivalent for performance and effectiveness to the ones dedicated to medical offices, being at the same time user-friendly and having an extremely convenient price in order to more spread the use of the magneto therapy by means of an user-friendly apparatus at a reasonable cost. A further object consists of combining the present invention with the applications of a smartphone.

These and also other objects, which will better appear in the following description, are substantially met by a magneto therapy apparatus according to what is disclosed in one or more of the attached claims and/or of the hereinbelow described characteristics, considered alone or in any combinations with each other or in combination with anyone of the attached claims.

According to a 1st independent aspect, it is provided a magneto therapy apparatus (1) comprising: at least one magneto therapy terminal (2) configured for and destined to be worn by a patient and for being positioned in proximity of a body surface of the patient, for performing a magneto therapy cycle, the terminal (2) being configured for generating at least one magnetic field, a control device (3) for supplying command signals to the magneto therapy terminal (2), and comprising at least one control unit (11) for supplying said command signals and an energy storage element (12) fixedly or removably connectable or connected to the magneto therapy terminal (2) for supplying an electric supply to said at least one magneto therapy terminal (2), the control device (3) comprising a wireless communication module (21) and being configured for wirelessly communicating with a mobile device (23).

According to a 2nd aspect dependent on the aspect 1, the energy storage element (12) is configured for operating at least between one operative condition wherein it is connected to the magneto therapy terminal (3) and supplies the terminal (3), the control unit (11) supplying command signals to the terminal (2) for performing a magneto therapy cycle and a recharge condition wherein is connected to an electric energy source for being recharged, particularly the energy storage element (12), under a recharge condition, being disconnected from the magneto therapy terminal (2).

According to a 3rd aspect dependent on anyone of the preceding aspects, the wireless communication module (21) is configured for transmitting information to, and receiving information from, a mobile device (23).

According to a 4th aspect dependent on anyone of the preceding aspects, the wireless communication module (21) comprises a Bluetooth module or an Internet module or a 3G or 4G module, or a Wi-Fi module.

According to a 5th aspect dependent on anyone of the preceding aspects, the control unit (11) is configured for supplying command electric signals to the terminal (2) for performing one or more magneto therapy cycles.

According to a 6th aspect dependent on anyone of the preceding aspects, the control unit (11) is configured for supplying command electric signals to the terminal (2) for managing the duration of the magneto therapy cycle and/or intensity, the frequency, the power and/or the duty cycle of the magnetic field generated by the terminal (2).

According to a 7th aspect dependent on anyone of the preceding aspects, the control unit (11) Is configured for supplying command electric signals to the terminal (2), the command electric signals comprising at least one signal indicative of the duration of the magneto therapy cycle.

According to an 8th aspect dependent on anyone of the preceding aspects, the control unit (11) is configured for sending command electric signals to the terminal (2), the command electric signals comprising at least one signal indicative of the duty cycle of the magnetic field generated by the terminal (2).

According to a 9th aspect dependent on anyone of the preceding aspects, the control unit (11) is configured for sending command electric signals to the terminal (2), the command electric signals comprising at least one signal indicative of the frequency of the magnetic field generated by the terminal (2).

According to a 10th aspect dependent on anyone of the preceding aspects, the apparatus further comprises a mobile device (23) operatively wirelessly connectable to the control unit (11) and configured for supplying command signals to the control unit (11) for performing one or more magneto therapy cycles.

According to an 11th aspect dependent on the preceding aspect, the mobile device (23) comprises a respective wireless communication module (22) configured for communicating with the wireless communication module (21) of the control device (3).

According to a 12th aspect dependent on the preceding aspect, the wireless communication module (21) of the control device (3) and the wireless communication module (22) of the mobile device (23) are of the same type.

According to a 13th aspect dependent on anyone of the preceding aspects 11 or 12, each wireless communication module (21, 22) comprises a Bluetooth module or an Internet module or a 3G or a 4G module, or a Wi-Fi Module.

According to a 14th aspect dependent on anyone of the preceding aspects 11 or 12 or 13, the mobile device (23) comprises a remote control unit operatively connected to said wireless communication module (22) and configured for supplying command signals to the control unit (11) of the control device (3) for managing the duration of the magneto therapy cycle and/or intensity, the frequency, power and/or duty cycle of the magnetic field generated by the terminal (2).

According to a 15th aspect dependent on anyone of the preceding aspects from 11 to 14, the mobile device (23) is configured for supplying command signals to the control unit (11) of the control device (3) in order to manage the execution of one or more magneto therapy cycles.

According to a 16th aspect dependent on anyone of the preceding aspects from 11 to 15, the mobile device (23) is configured for supplying command signals to the control unit (11) of the control device (3) by a mobile application or software, said mobile application or said software operating and/or being installed on the mobile device (23) and/or being executable by the mobile device (23) by a connection to a computer.

According to a 17th aspect dependent on the preceding aspect, said mobile application or said software is configured for wirelessly communicating with the control unit (11) of the control device (3) for setting, selecting and/or managing one or more magneto therapy cycles performed by the terminal (2).

According to an 18th aspect dependent on anyone of the preceding aspects from 11 to 17, the mobile device (23) comprises a memory configured for storing the magneto therapy cycles performed by the terminal (2) in order to provide on the mobile device (23) a record of the performed magneto therapy cycles.

According to a 19th aspect dependent on anyone of the preceding aspects from 11 to 18, the mobile device (23) comprises a user interface adapted to and configured for managing one or more magneto therapy cycles performed by the terminal (2).

According to a 20th aspect dependent on anyone of the preceding aspects from 11 to 19, the mobile device (23) comprises a cellphone or a smartphone or laptop computer or a Personal Digital Assistant or a tablet.

According to a 21st aspect dependent on anyone of the preceding aspects, the control unit (11) comprises at least one card (13) provided with a first inlet (14) for being connected to an electric energy source and with at least one second inlet (15) for being connected to the magneto therapy terminal (2).

According to a 22nd aspect dependent on the preceding aspect, the card (13) comprises a plurality of second inlets (15), each of them is fixedly or removably connectable or connected to a respective magneto therapy terminal (2).

According to a 23rd aspect dependent on anyone of the preceding aspects, the control unit (11) of the control device (3) is configured for wirelessly supplying to the mobile device (23) information regarding the magneto therapy cycle performed by the terminal (2).

According to a 24th aspect dependent on anyone of the preceding aspects from 10 to 23, the mobile device (23) is configured for providing information or parameters regarding the magneto therapy cycle performed by the terminal (2).

According to a 25th aspect dependent on anyone of the preceding aspects, the magneto therapy terminal (2) comprises an inlet (10) to be connected to the energy storage element (12).

According to a 26th aspect dependent on anyone of the preceding aspects, the apparatus comprises a recharge connector (16) adapted to enable to connect the energy storage element (12) to an electric energy source.

According to a 27th aspect dependent on the preceding aspect, the recharge connector (16) is of a USB type and/or comprises a transformer.

According to a 28th aspect dependent on anyone of the preceding aspects, the apparatus comprises a connection member (18) adapted to enable to connect the energy storage element (12) to the terminal (2).

According to a 29th aspect dependent on anyone of the preceding aspects, the magneto therapy terminal (2) exhibits a substantially annular shape.

According to a 30th aspect dependent on anyone of the preceding aspects, the magneto therapy terminal (2) comprises a casing (6) and a magnetic field generator (7) housed inside the casing (6).

According to a 31st aspect dependent on the preceding aspect, the casing (6) is made of a magnet field permeable material, for example of plastics.

According to a 32nd aspect dependent on anyone of the preceding aspects from 30 to 31, the casing (6) is of a sanitizable material.

According to a 33rd aspect dependent on anyone of the preceding aspect 30 or 31 or 32, the magnetic field generator (7) is configured for generating at least one magnetic field having a variable intensity, frequency and/or power.

According to a 34th aspect dependent on anyone of the preceding aspects from 30 to 33, wherein the magnetic field generator (7) exhibits a toroidal shape, particularly defined by a coil (9) wound around the toroid axis.

According to a 35th aspect dependent on anyone of the preceding aspects from 30 to 34, wherein the magnetic field generator (7) comprises a coil (9) having a substantially annular development.

According to a 36th aspect dependent on the preceding aspect, the magnetic field generator (7) comprises a coil coated by a plastic material, for example of a type destined to a medical use (8).

According to a 37th aspect dependent on anyone of the preceding aspects, the apparatus comprises at least one elastic band (4) configured for enabling to stably position said at least one magneto therapy terminal (2) at a body surface of the patient, destined to be treated.

According to a 38th aspect dependent on the preceding aspect, the elastic band (4) is configured for housing the control device (3).

According to a 39th aspect dependent on anyone of the preceding aspects, the control device (3) is devoid of a screen displaying information regarding a magneto therapy cycle performed by the terminal (2).

According to a 40th aspect dependent on anyone of the preceding aspects, the control device (3) is devoid of a data cable or a data connection configured for being connected to a remote unit.

According to a 41st aspect dependent on anyone of the preceding aspects, the control device (3) exhibits a height (H3) comprised between 80 mm and 150 mm, particularly comprised between 100 mm and 130 mm.

According to a 42nd aspect dependent on anyone of the preceding aspects, the control device (3) exhibits a width (L3) comprised between 40 mm and 90 mm, particularly comprised between 60 mm and 75 mm.

According to a 43rd aspect dependent on anyone of the preceding aspects, the control device (3) exhibits a thickness (S3) comprised between 10 mm and 30 mm, particularly comprised between 10 mm and 20 mm.

According to a 44th aspect dependent on anyone of the preceding aspects, the weight of the control device (3) is comprised between 50 g and 200 g, particularly is comprised between 50 g and 100 g.

According to a 45th aspect dependent on anyone of the preceding aspects, the energy storage element (12) comprises a battery, particularly a lithium-ion battery.

According to a 46th aspect dependent on anyone of the preceding aspects, the control device (3) comprises one or more indicators (17) of the residual energy level of the energy storage element and/or of an operative condition of the energy storage element (12).

According to a 47th aspect dependent on the preceding aspect, the indicators (17) comprise one or more LED, particularly an amber LED and at least one green LED.

According to a 48th aspect dependent on anyone of the preceding aspects, the control device (3) comprises on/off means (19).

According to a 49th aspect dependent on the preceding aspect, the on/off means (19) comprise at least one on/off button or are of a touch-type.

According to a 50th aspect dependent on anyone of the preceding aspects, the control device (3) comprises means (20) for pausing/playing the execution of a magneto therapy cycle.

According to a 51st aspect dependent on the preceding aspect, the pause/play means (20) comprise at least one pause/play button or are of a touch-type.

According to a 52nd aspect dependent on anyone of the preceding aspects, the apparatus is configured for storing one or more therapy cycles performed by the patient and for sharing them by email or by a mobile application or by a software suitable for a personal computer.

According to a 53rd aspect dependent on anyone of the preceding aspects, the apparatus is configured for performing a pain test and for storing data regarding the test result for being capable of tracking the possible progress or regress of the pathology and enabling a caring physician both to verify and possibly modify one or more suggested magneto therapy cycles.

According to a 54th independent aspect, it is provided an electrotherapy apparatus, particularly for magneto therapy, electrostimulation, laser therapy and tecar therapy, comprising: at least one electrotherapy terminal (2) configured for and destined to be worn by a patient and/or for being positioned in proximity of a body surface of the patient for performing an electrotherapy cycle, a control device (3) for supplying command signals to the electrotherapy terminal (2) and comprising at least one control unit (11) for supplying said command signals and an energy storage element (12) fixedly or removably connectable or connected to the electrotherapy terminal (2) for supplying an electric supply to said at least one electrotherapy terminal (2), the control device (3) comprising a wireless communication module (21) and being configured for wirelessly communicating with a mobile device (23).

According to a 55th aspect dependent on the preceding aspect, the energy storage element (12) is configured for operating at least between an operative condition wherein is connected to the electrotherapy terminal (3) and supplies the terminal (3), the control unit (11) supplying command signals to the terminal (2) or performing an electrotherapy cycle, and a recharge condition wherein is connected to an electric energy source for being recharged, particularly the energy storage element (12) under a recharge condition, being disconnected from the electrotherapy terminal (2).

According to a 56th aspect dependent on the preceding aspect 54 or 55, the wireless communication module (21) is configured for transmitting information to, and receiving information from, a mobile device (23).

According to a 57th aspect dependent on anyone of the preceding aspects from 54 to 56, the wireless communication module (21) comprises a Bluetooth module or an Internet module or a 3G or 4G module, or a Wi-Fi module.

According to a 58th aspect dependent on anyone of the preceding aspects from 54 to 57, the control unit (11) is configured for supplying command electric signals to the terminal (2) for performing one or more electrotherapy cycles.

According to a 59th aspect dependent on anyone of the preceding aspects, the apparatus further comprises a mobile device (23) operatively wirelessly connectable to the control unit (11) and configured for supplying command signals to the control unit (11) for performing one or more electrotherapy cycles.

According to a 60th aspect dependent on the preceding aspect, the mobile device (23) comprises a respective wireless communication module (22) configured for communicating with the wireless communication module (21) of the control device (3).

According to a 61st aspect dependent on the preceding aspect, the wireless communication module (21) of the control device (3) and the wireless communication module (22) of the mobile device (23) are of the same type.

According to a 62nd aspect dependent on anyone of the preceding aspects from 60 to 61, each wireless communication module (21, 22) comprises a Bluetooth module or an Internet module or a 3G or 4G module, or a Wi-Fi module.

According to a 63rd aspect dependent on anyone of the preceding aspects 60 or 61 or 62, the mobile device (23) comprises a remote control unit operatively connected to said wireless communication module (22) and configured for supplying command signals to the control unit (11) of the control device (3) for managing the duration of the electrotherapy cycles, and/or operative parameters of the electrotherapy cycles such as the intensity, frequency, power and/or the duty cycles.

According to a 64th aspect dependent on anyone of the preceding aspects from 60 to 63, the mobile device (23) is configured for supplying command signals to the control unit (11) of the control device (3) for managing the execution of one or more electrotherapy cycles.

According to a 65th aspect dependent on anyone of the preceding aspects from 60 to 64, wherein the mobile device (23) is configured for supplying command signals to the control unit (11) of the control device (3) by a mobile application or software, said mobile application or said software operating and/or being installed in the mobile device (23) and/or being executable by the mobile device (23) through a connection to a computer.

According to a 66th aspect dependent on the preceding aspect, said mobile application or said software is configured for wirelessly communicating with the control unit (11) of the control device (3) for setting, selecting and/or managing one or more electrotherapy cycles performed by the terminal (2).

According to a 67th aspect dependent on anyone of the preceding aspects from 60 to 66, wherein the mobile device (23) comprises a memory configured for storing the electrotherapy cycles performed by the terminal (2) for providing on the mobile device (23) a record of the performed electrotherapy cycles.

According to a 68th aspect dependent on anyone of the preceding aspects from 60 to 67, wherein the mobile device (23) comprises a cellphone or smartphone or laptop computer or a Personal Digital Assistant or a tablet.

The detailed description of one or more preferred embodiments of the invention are given in an exemplifying and non-limiting way, wherein:
Figure 1 illustrates a magneto therapy apparatus according to the present invention;
Figure 2 illustrates the control device of a magneto therapy apparatus according to the present invention;
Figures 3a and 3b illustrate a magneto therapy apparatus according to the present invention under operative conditions;
Figure 4 represents the magnetic field generator of the terminal of a magneto therapy apparatus according to the present invention.

With reference to the figures, 1 generally indicates a magneto therapy apparatus according to the present invention.

As discussed, the devices are destined to be used for the magneto therapy (PEMF, CMP, low frequency pulsed magneto therapy) for accelerating repairing processes (particularly with reference to the bones) or for obtaining analgesic effects.

The magneto therapy of the present invention is particularly useful for a plurality of pathologies as exemplifyingly described in the following.

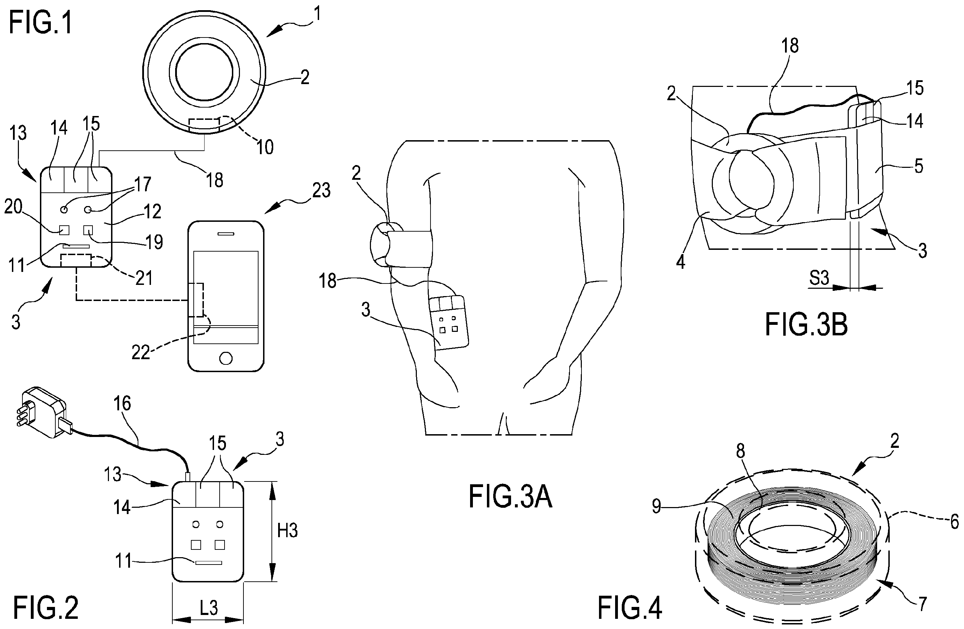

The apparatus 1 comprises a magneto therapy terminal 2 and a control device 3 of the terminal 2. The terminal 2 is the element of the apparatus 1, which is positioned at the body surface of the patient to be treated (see Figures 3a and 3b) for achieving the desired therapeutic effect. The terminal 2 can be stably positioned adjacent a body area of the patient by one or more suitable elastic bands 4. In other words, the elastic bands 4 enable the patient to comfortably wear the terminal 4 for being capable of performing the therapy. Figures 3a and 3b illustrate, for example, how to position the terminal 2 in proximity of the right elbow of the patient. Advantageously, the elastic band 4 can comprise a pocket 5 enabling to house the control device 3 (see Figure 3b).

As illustrated in Figure 1, the terminal 2 exhibits a substantially annular development and comprises an outer casing 6 made of a material adapted to get in contact with body surfaces of the patient. The casing 6 can be made of a plastic material and is adapted to not overheat under the operative conditions of the apparatus 1. The material of the casing 6 can be sanitizable for ensuring a greater hygiene of the terminal 2. For example, under instances wherein the terminal 2 is used by a plurality of patients, it can be advantageous to sanitize or disinfect the casing itself of the terminal 2 after using the apparatus 1. Further, the material of the casing 6 is permeable to magnetic fields, so that the magnetic fields can cross it.

The casing 6 houses inside a magnetic field generator 7, which is schematically illustrated in Figure 4. The magnetic field generator 7 is configured for generating, at command of the control device 3 (as it will be more specifically described hereinelow), at least one magnetic field. The magnetic fields generated by the generator 7, and generally by the apparatus 1, can be used for the therapy of at least one of the following diseases: arthritis, arthropaties, arthrosis, muscular atrophies, bursitises, cervicalgia, whiplashes, contractures, contusions, coxarthrosis, rotator cuffs, decontracting therapy, distortions, back pain, articular pains, epicondylitis, epitrocleitis, fractures, backache, abarticulations, intramuscular fibrositis, osteoporosis, periarthritis, rehabilitation following reconstruction of the anterior cruciate ligament, post, pseudoarthrosis, cartilage cleaning, consolidation delays, muscle lacerations, incomplete dislocations, tendinitises, tendinous synovitises, torticollis.

The magnetic field generator 7 exhibits a substantially toroidal shape, as illustrated in Figure 4, and comprises a support 8 having an annular shape, around which one (or more) coils 9 are wound (see Figure 4).

The toroidal shape of the magnetic field generator 7 is particularly advantageous because amplifies the power of the magnetic field inside it and enables to promote the density of the energy of the magnetic field in the desired point. Moreover, the structure of the magnetic field generator 7 enables to eliminate the excess heat which forms during the generation of the magnetic field. In this way, overheating the casing 6 is avoided and the patient is not subjected to uncomfortable and undesired sensations of localized heat.

Further, the terminal 2 comprises (at least) one inlet 10 configured for enabling the connection to the control device 3.

The apparatus 1 can comprise two or more terminals 2 of the above described type, which are commanded by the same control device 3 for performing one or more magneto therapy cycles.

De facto, for some applications, it can be necessary to use and position more than one terminal at the area to be treated in order to maximize the therapeutic effect.

The control device 3 comprises a control unit 11 and an energy storage element (or battery) 12. The control unit 11 is in charge of managing the operation of the control device 3 and terminal; it comprises a card 13 provided with a first inlet 14 to be connected to an electric energy source and to be connected to a personal computer, at least one second inlet 15 to be connected to the terminal 2.

The first inlet 14 enables to recharge the energy storage element 12, which is performed by connecting the first inlet 14 to an electric energy source. Connecting the energy storage element 12 to the electric energy source is done by a recharge connector 16. As a function of the type of the electric energy source, the recharge connector 16 can take different shapes. For example, when the electric energy source is a wall outlet, the recharge connector 16 can be of a type comprising a transformer. Alternatively, the electric energy source can be implemented by a mobile device such as a laptop computer or a personal computer and the recharge connector 16 can be implemented by an USB cable. Preferably, under recharge conditions, the energy storage element 12 is disconnected from the magneto therapy terminal 2 and therefore the apparatus 1 cannot be used for performing a magneto therapy cycle.

The energy storage element 12 can consist of a lithium-ion battery enabling to reduce the weight of the control device 3, ensuring at the same time a good operative autonomy of the control device 3. For example, the autonomy of the storage element 13 can be up to a maximum of three hours, and enables the charged storage element 12 to supply the electric energy necessary for the terminal 2 to perform at least one magneto therapy cycle. Generally, the autonomy of the energy storage element 12 varies between one hour and three hours, as a function of the magneto therapy cycles performed by the apparatus 1. Moreover, the control device 3 comprises one or more residual energy level indicators 17 of the energy storage element. The indicators 17 can comprise one or more LEDs adapted to supply a visual warning of the residual energy level. Particularly, the indicators 17 can comprise an amber LED which can be adapted at least to signal a discharge condition of the energy storage element 12, and a green LED adapted to signal a charge condition of the energy storage element. Alternatively, the green LED can be instrumental in signaling the operative condition of the apparatus 1 (the magneto therapy cycle is in progress).

The second inlet 15 of the card 13 enables the connection of the energy storage element 12 to the terminal 2. The control device 3 can be configured for being connected to a plurality of terminals 2; such connection can be managed by a single second inlet 15 or by a plurality of second inlets 15 each of them is configured for being connected to a respective terminal 2. For example, Figure 1 illustrates a card 13 having two second inlets 15, each of them is configured for being connected to a respective terminal 2.

Under operative conditions of the apparatus 1, the control device 3 is connected to the inlet of the terminal 2, for supplying to it the electric supply and particularly for supplying electric command signals necessary for generating the magnetic field by the magnetic field generator 7.

According to a preferred embodiment of the invention, the control device 3 is removably connectable to the magneto therapy terminal 2 by a connection member 18, which can consist in a cable. The connection member 18 can be selectively connected and disconnected from the terminal 2 and energy storage element 12.

Supplying the electric command signals to the terminal 2 is managed by the control unit 11 of the control device 3. The control unit 11 supplies electric command signals enabling to manage the duration of the magneto therapy cycle and the duty cycle of the magnetic field generated by the magnetic field generator 7. Moreover, the electric command signals supplied to the control unit 11 are capable of managing the frequency, intensity of the magnetic field generated by the magnetic field generator 7. In other words, the control unit 11 of the control device 3 supplies the coil of the magnetic field generator 7 by electric pulses (command signals) adapted to generate magnetic fields having specific parameters enabling to follow determined treatment protocols and treat determined pathologies of the patient.

The control device 3 exhibits a limited size and therefore is very compact; such characteristics make very easy and practical using the control device 3 and minimize the volume of the apparatus 1.

The control device 3 can exhibit, for example, a height H3 comprised between 80 mm and 150 mm, particularly comprised between 100 mm and 130 mm. The width L3 of the control device can be comprised between 40 mm and 90 mm and particularly is comprised between 60 mm and 75 mm. Moreover, the control device 3 is very thin; the thickness of the control device 3 can be comprised between 10 mm and 30 mm, particularly is comprised between 10 mm and 20 mm.

Further, the control device 3 is very light; indeed, it can weight between 50 g and 200 g, particularly between 50 g and 100 g. The limited size and weight of the control device 3 enable an excellent portability. Under operative conditions of the apparatus 1, indeed the control device can be worn by the patient himself/herself, for example by means of the elastic band 4 (see Figure 3b).

In addition, the control device 3 comprises on/off means 19 configured for turning on/off the control device 3 itself. The on/off means 19 can be touch sensitive, for example of a touch-type, or can comprise an on/off button.

Pausing or playing a magneto therapy cycle can be managed by the control device 3 so that it can comprise pause/play means 20. The means 20 for pausing/playing a magneto therapy cycle in turn can be touch sensitive, or can comprise a pause/play button.

The control device 3 is not provided with any screen adapted to display information regarding the magneto therapy cycle performed by the terminal 2. The information and parameters of the magneto therapy cycle can be displayed by a different mobile device, which will be more particularly described in the following.

Moreover, the control device 3 comprises a wireless communication module 21 configured for communicating with a wireless communication module 22 of a mobile device 23. The wireless communication modules 21, 22 can be of the same type and can communicate by a Bluetooth or Internet or 3G or 4G or Wi-Fi technology. Specifically, the wireless communication modules 21, 22 can be of a Bluetooth 4.0 type, also called *Bluetooth LE* (*Bluetooth Low Emission* or *Bluetooth Low Energy*)*.*

The wireless communication module 21 of the control device 3 can both receive command signals from the mobile 23 and supply information to the mobile device 23. In other words, the control device 3 and mobile device 23 wirelessly communicate and therefore the control device 3 does not require a data cable. The information supplied by the control device 3 to the mobile device 23, are for example in relation with the condition of the magneto therapy cycle (for example the cycle in progress or the paused or interrupted cycle) or in relation to the remaining time to the end of the magneto therapy cycle.

Moreover, the mobile device 23 can comprise a control unit operatively connected to the wireless communication module 22. The control unit of the mobile device 23 manages the interaction and information exchange between the mobile device 23 and control device 3 and commands to play, pause, and resume a magneto therapy cycle.

Operating the control device 3 and commanding the magneto therapy cycle are performed by a mobile application or software running in the mobile device 23. According to a variant, managing the operation of the control device 3 can be performed by accessing to a web site from the mobile device 23 by the Internet network.

The mobile device 23 can consist in a cellphone or smartphone or a laptop computer or a Personal Digital Assistant or a tablet. Using a dedicated application or software operating in the mobile device 23 is particularly advantageous because enables to use the mobile device 23 for managing the magneto therapy treatments, even though the mobile device 23 is not specifically destined to the magneto therapy. Moreover, the magneto therapy cycles can be directly managed by the patient through the mobile device 23, because the user interface of the application or software is very simple and user friendly.

The control device 3 can comprise a memory storing at least one magneto therapy program and therefore can operate in a way completely independent, or can be commanded by the mobile device 23 or personal computer.

Some of the available functionalities and that can be managed by the mobile device 23 will be described in the following with a particular reference to an application. Such functionalities and characteristics can be implemented analogously by a software.

First of all, the application enables to select, by the mobile device 23, a magneto therapy cycle or program. Indeed, the application is provided with predefined programs, to each of them a minimum and/or maximum duration and associated intervals between magneto therapy treatments under generic conditions are associated. The caring physician, obviously, after evaluating the specific conditions of the patient, the stage of the disease or other variables, can prescribe different timings.

Moreover, the application enables the qualified personal to modify the therapy and/or generate a protocol specific for the pathology of each patient. Generating and saving a specific magneto therapy cycle (program) could be done both by an application directly to the mobile device 23 and through a software by a laptop or personal computer, for being then loaded in the mobile device 23. The generation of a customized extended magneto therapy cycle can be done only by a laptop or personal computer.

The application or software for a laptop or a personal computer enables therefore the physicians to offer their patients more specific treatment programs and to track and analyze by a (remote) computer the trend of the activity of the patient in relation with the use of the control device 3 and the felt pain. According to an operative mode of the application, the qualified physician or a technician assigned by a practitioner, can select the therapy from a list of programs (also by combining programs with customized programs) and makes it available in the mobile device 23 of the patient with the quantity or according to modes selected by the physician.

Moreover, the application enables to store in a memory the magneto therapy cycles performed by a single patient and makes available the record of the therapies performed by the patient in order to evaluate the encountered improvements.

Upon starting the application, the mobile device 23 synchronizes with and establishes a connection with the command device 3 by means of the wireless communication modules 21, 22.

The application enables to manage the therapies of a plurality of patients by the same mobile device 23. Upon starting the application, de facto it can provide the selection of an user profile. After this first selection, the application enables to select a magneto therapy cycle adapted to the pathology of the patient.

Moreover, the application enables to manage over time the effectiveness of the magneto therapy treatment. De facto, the application enables to evaluate the level of the pain felt by the patient-user by a self-evaluation scale (visual-analgesic scale or VAS) on a plurality of levels (four levels, for example). Both the patient and physician are therefore capable of plotting over time the symptomatology and therefore the effectiveness of the treatment, enabling the qualified personnel to modify the therapy. Before the treatment, and after selecting the program to run, the user interface will show a display with four icons representing four intensities of the felt pain (no pain, small pain, much pain, a very strong pain). At this time, it is required to select the icon representing the sensation of the pain felt in this moment. Therefore, it is possible to save such setting; the test will be stored and the initial display will appear from which the therapy start button could be selected.

The present invention enables to obtain one or more of the following advantages and to solve one or more of the problems found in the prior art.

First of all, the invention enables to provide a portable magneto therapy apparatus which can be used by the patient also when he/she performs other activities because it is provided with a battery and is extremely light and small.

Controlling and managing the control device by the interaction between the control device and mobile device enables to perform magneto therapy cycles without compelling the patient to go to a physiotherapy or another specialized centre.

The invention enables to control and manage the magneto therapy treatments by a smartphone or personal computer, enabling to easily and intuitively manage the therapy.

Moreover, the present invention enables to customize and adapt the magneto therapy cycles based on the preferences of the user and/or on the physical conditions thereof.

The invention enables the patients to be comfortably subjected to magneto therapy cycles at home and remotely assisted by a physician.

Moreover, the invention enables to promote the diffusion of magneto therapy apparatuses, because of the reasonable cost and user-friendliness of the apparatus.

In addition, the invention has a convenient use, is easy to implement and can be simply and economically manufacturable.

## Claims

1. Magneto therapy apparatus (1) comprising:
at least one magneto therapy terminal (2) configured for and destined to be worn by a patient and for being placed in proximity of a body surface of the patient for performing a magneto therapy cycle, the terminal (2) being configured for generating at least one magnetic field,
a control device (3) for supplying command signals to the magneto therapy terminal (2) and comprising at least one control unit (11) for supplying said command signals and an energy storage element (12) fixedly or removably connectable or connected to the magneto therapy terminal (2) for supplying an electric supply to said at least one magneto therapy terminal (2), the control device (3) comprising a wireless communication module (21) and being configured for wirelessly communicating with a mobile device (23).

2. Apparatus according to claim 1, wherein the energy storage element (12) is configured for operating at least between an operative condition wherein it is connected to the magneto therapy terminal (3) and supplies the terminal (3), the control unit (11) supplying command signals to the terminal (2) for performing a magneto therapy cycle, and a recharge condition wherein it is connected to an electric energy source for being recharged, particularly the energy storage element (12) under the recharge condition, being disconnected from the magneto therapy terminal (2) and not connectable to the same.

3. Apparatus according to claim 1 or 2, wherein the wireless communication module (21) is configured for transmitting information to, and receiving information from, a mobile device (23) and comprises a Bluetooth module or an Internet module or a 3G or 4G module or a Wi-Fi module.

4. Apparatus according to anyone of the preceding claims, wherein the control unit (11) is configured for supplying command electric signals to the terminal (2) for performing one or more magneto therapy cycles, the command electric signals comprising at least one signal indicative of the duration of the magneto therapy cycle and/or a signal indicative of the duty cycle of the magnetic field generated by the terminal (2) and/or at least one signal indicative of the frequency of the magnetic field generated by the terminal (2).

5. Apparatus according to anyone of the preceding claims, further comprising a mobile device (23) operatively wirelessly connectable to the control unit (11) and configured for supplying command signals to the control unit (11) for performing one or more magneto therapy cycles, the mobile device (23) comprising a respective wireless communication module (22) configured for communicating with the wireless communication module (21) of the control device (3), particularly the mobile device (23) comprising a cellular phone or smartphone or laptop computer or a Personal Digital Assistant or a tablet.

6. Apparatus according to the preceding claim, wherein the wireless communication (21) of the control device (3) and the wireless communication module (22) of the mobile device (23) are of the same type, for example a Bluetooth module or an Internet module or a 3G or 4G module, or a Wi-Fi module.

7. Apparatus according to the preceding claim 5 or 6, wherein the mobile device (23) comprises a memory configured for storing the electrotherapy cycles performed by the terminal (2) in order to provide a record of the performed electrotherapy cycles on the mobile device (23).

8. Apparatus according to anyone of claims from 5 to 7, wherein the mobile device (23) is configured for supplying command signals to the control unit (11) of the control device (3) by a mobile application or software, said mobile application or said software operating and/or being installed on the mobile device (23) and/or being executable by the mobile device (23) by a connection to a computer, and wherein said mobile application or said software is configured for wirelessly communicating with the control unit (11) of the control device (3) for setting, selecting and/or managing one or more magneto therapy cycles performed by the terminal (2), and comprises an user interface adapted and configured for managing one or more magneto therapy cycles performed by the terminal (2).

9. Apparatus according to anyone of the preceding claims, wherein the control unit (11) comprises at least one card (13) provided with a first inlet (14) for being connected to an electric energy source and a plurality of second inlets (15), each of them is fixedly or removably connectable or connected to a respective magneto therapy terminal (2), and wherein the magneto therapy terminal (2) comprises an inlet (10) for the connection to the energy storage element (12).

10. Apparatus according to anyone of the preceding claims, wherein the magneto therapy terminal (2) exhibits a substantially annular shape and comprises a casing (6) of magnetic field permeable material, for example of a sanitizable plastic, and a magnetic field generator (7) housed inside the casing (6), the magnetic field generator (7) exhibiting a toroidal shape, particularly defined by a coil (9) having a substantially annular development wound around the axis of the toroid and being configured for generating at least one magnetic field having a variable intensity, frequency and/or power.

11. Apparatus according to anyone of the preceding claims, comprising at least one elastic band (4) configured for enabling to stably position said at least one magneto therapy terminal (2) at a body surface of the patient destined to be treated, the elastic band (4) being optionally configured for housing the control device (3).

12. Apparatus according to anyone of the preceding claims, wherein the control device (3) is devoid of a screen displaying information regarding the magneto therapy cycle performed by the terminal (2), and is devoid of a data cable or data connection configured for being connected to a remote unit different from the magneto therapy terminal.

13. Apparatus according to anyone of the preceding claims, wherein the control device (3) exhibits:
- a height (H3) comprised between 80 mm and 150 mm, particularly comprised between 100 mm and 130 mm;
- a width (L3) comprised between 40 mm and 90 mm, particularly comprised between 60 mm and 75 mm;
- a thickness (S3) comprised between 10 mm and 30 mm, particularly comprised between 10 mm and 20 mm;
the weight of the control device (3) being comprised between 50 g and 200 g, particularly comprised between 50 g and 100 g.

14. Apparatus according to anyone of the preceding claims, wherein the control device (3) comprises:
- one or more indicators (17) of the residual energy level of the energy storage element and/or of an operative condition of the energy storage element (12), particularly the indicators (17) comprise one or more LEDs;
- on/off means (19) with at least one on/off button or of a touch-type;
- means (20) for pausing/playing the execution of a magneto therapy cycle, particularly comprising at least one pause/play button or being of a touch-type.

15. Apparatus according to anyone of the preceding claims, configured for performing a pain test and storing data regarding the result of the test in order to follow the possible advancement or regression of the disease and for enabling to verify and possibly modifying one or more suggested magneto therapy cycles.
